# FASCICULE DE BREVET EUROPEEN

(11) **EP 3 293 518 B1**
(45) Date de publication et mention de la délivrance du brevet: **01.12.2021**
(21) Numéro de dépôt: 17190623.3
(22) Date de dépôt: 12.09.2017
(51) Int. Cl.: G01N 33/00, G01N 29/02, G01N 29/036

(54) **CAPTEUR DE H2S A ONDE ELASTIQUE COMPORTANT UN FILM DE POLYMERE COMPRENANT DES FONCTIONS CARBOXYLATES ET DES CATIONS DE PLOMB OU DE ZINC ET PROCEDE DE FABRICATION**
AKUSTISCHER H2S-SENSOR MIT EINER POLYMERSCHICHT MIT CARBOXYLATGRUPPEN UND BLEI- ODER ZINKKATIONEN, UND ENTSPRECHENDES HERSTELLUNGSVERFAHREN
ELASTIC WAVE H2S SENSOR INCLUDING A POLYMER FILM COMPRISING CARBOXYLATE GROUPS AND LEAD OR ZINC CATIONS, AND MANUFACTURING METHOD

(30) Priorité: 13.09.2016 FR 1658487
(43) Date de publication de la demande: 14.03.2018
(73) Titulaire: Senseor, 06560 Valbonne (FR); CENTRE NATIONAL DE LA RECHERCHE SCIENTIFIQUE, 75016 Paris (FR); TOTAL SE, 92400 Courbevoie (FR); Université de Franche-Comté, 25030 Besançon Cedex (FR)
(72) Inventeur: FRIEDT, Jean-Michel, 25000 Besancon (FR); CHERIOUX, Frédéric, 25720 Larnod (FR); LAMARE, Simon, 58660 Coulanges Les Nevers (FR); GEGOT, François, 25000 Besancon (FR)
(74) Mandataire: Marks & Clerk France

(56) Documents cités:
- US-A- 3 167 734
- US-A- 5 034 452
- HANMING DING ET AL: "Detection of hydrogen sulfide: the role of fatty acid salt Langmuir-Blodgett films", MATERIALS SCIENCE AND ENGINEERING C., vol. 11, no. 2, 28 novembre 2000 (2000-11-28), pages 121-128, XP055363717, CH ISSN: 0928-4931, DOI: 10.1016/S0928-4931(00)00195-8
- G I DZHARDIMALIEVA ET AL: "Macromolecular metal carboxylates", RUSSIAN CHEMICAL REVIEWS (USPEKHI KHIMII)., vol. 77, no. 3, 31 mars 2008 (2008-03-31), pages 259-301, XP055364214, GB ISSN: 0036-021X, DOI: 10.1070/RC2008v077n03ABEH003682
- Zainab Yunusa ET AL: "Gas Sensors: A Review", Sensors & Transducers, 30 avril 2014 (2014-04-30), pages 61-75, XP055363935, Extrait de l'Internet: URL:http://www.sensorsportal.com/HTML/DIGE ST/april_2014/Vol_168/P_1957.pdf [extrait le 2017-04-11]
- W P JAKUBIK ET AL: "POLYANILINE THIN FILMS AS A TOXIC GAS SENSORS IN SAW SYSTEM", MOLECULAR AND QUANTUM ACOUSTICS, vol. 28, 2007, page 125, XP055446416,
- ASAD MOHSEN ET AL: "Surface acoustic wave based H2S gas sensors incorporating sensitive layers of single wall carbon nanotubes decorated with Cu nanoparticles", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 198, 18 mars 2014 (2014-03-18), pages 134-141, XP028658237, ISSN: 0925-4005, DOI: 10.1016/J.SNB.2014.03.024

## Description

Le domaine de l'invention est celui de la détection de sulfure d'hydrogène présent en phase gazeuse ou liquide et considéré dans sa première forme comme toxique pour des concentrations de l'ordre de 20 ppm. Produit notamment par des bactéries contaminant les forages pétroliers, sa détection peut représenter un enjeu majeur.

L'art connu dans ce domaine, propose un certain nombre de solutions (électrochimiques, colorimétriques, conductimétriques) incompatibles avec le déploiement de capteur passif, autonome et pouvant être interrogé à distance sans liaison filaire, par exemple par une liaison radiofréquence.

Dans ce contexte, les Demandeurs ont cherché une solution permettant notamment de fonctionnaliser un transducteur piézoélectrique à onde élastique permettant d'élaborer un capteur compatible avec l'absence de source locale d'énergie et avec des mesures à distance sans fil.

Les demandeurs ont ainsi élaboré un film de matériau sensible au H₂S pouvant être réalisé de manière reproductible, et d'épaisseur contrôlée, pouvant notamment être utilisé dans un capteur passif propageant une onde élastique permettant la détection de présence de H₂S sous la forme d'une masse de gaz absorbée sur la surface.

De manière extensive, un film de matériau sensible d'épaisseur contrôlée peut être exploité avec tout transducteur à détection directe (par exemple optique ou par résonance plasmon de surface).

De manière générale, il est déjà connu des capteurs passifs à onde élastique interrogeables à distance comprenant une couche sensible permettant de détecter certains types de composés. La masse captée est en mesure d'induire une variation des conditions limites de propagation de l'onde élastique, classiquement il s'agit d'un ralentissement de l'onde, observée comme une décroissance de la phase du signal radiofréquence excitant le transducteur.

Dans le contexte de la présente invention, les Demandeurs ont notamment conçu un capteur passif utilisant un transducteur à onde élastique de l'art connu et l'ont fonctionnalisé avec une couche contenant des sites réactifs afin d'ajouter au transducteur classique, une sensibilité spécifique à H₂S.

Pour ce faire, les Demandeurs ont recherché à exploiter un composé connu pour sa réaction sélective avec l'élément soufre. Des éléments tels que les ions de zinc, cuivre, or ou plomb sont connus pour réagir spécifiquement avec le soufre. Cependant, le cuivre présente une réaction défavorable avec l'oxygène pour former de l'oxyde de cuivre, tandis que l'or s'avère potentiellement complexe à manipuler.

Un mode connu pour de détection du H₂S consiste à utiliser un papier imbibé d'acétate de plomb, Pb(CH₃COO)₂ et est décrit dans l'article de T. Ding, J.-R. Zhang, S. Long, and J.-J. Zhu. Synthesis of HgS and PbS nanocrystals in a polyol solvent by microwave heating. Microelectronic Engineering, 66 :46-52, 2003. Dans ce cas, la molécule ne présente pas la capacité à être polymérisée et ne fait qu'imprégner le papier.

Un mode de production de ce composé est la réaction d'un cation Pb(II) avec l'acide acétique en milieu basique.

La réaction du produit ainsi formé avec H₂S induit la transformation en sulfure de Pb(II), PbS, un précipité gris, selon la réaction :

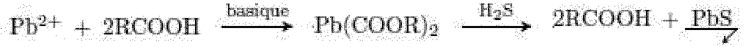

L'affinité du soufre pour les cations Pb(ll) est très forte. Elle permet, par exemple, de former des nano-particules de PbS à partir de soufre solide, d'anions sulfure ou de H₂S, comme décrit dans les articles de T. Ding, J.-R. Zhang, S. Long, and J.-J. Zhu. Synthesis of HgS and PbS nanocrystals in a polyol solvent by microwave heating. Microelectronic Engineering, 66 : 46-52, 2003 et de H. Karami, M. Ghasemi, and S. Matini. Synthesis, characterization and application of lead sulfide nano- structures as ammonia gas sensing agent. Int. J. Electrochem. Sci., 8 : 11661-11679, 2013 et de Z. Zeng, S. Wang, and S. Yang. Synthesis and characterization of PbS nanocrystallites in random copolymer ionomers. Chem. Mater., 11 :3365-3369, 1999.

Un autre mode connu pour détecter du H2S consiste à utiliser un film des sels d'acides gras de zinc ou de plomb et est décrit dans l'article HANMING DING ET AL: "Détection of hydrogen sulfide: the role of fatty acid salt Langmuir-Blodgett films", MATERIALS SCIENCE AND ENGINEERING C., vol. 11, no. 2, 28 novembre 2000, pages 121-128, XP055363717, DOI: 10.1016/ S0928-4931(00)00195-8.

Plus précisément, pour pouvoir notamment réaliser un capteur de type capteur passif à onde élastique, les Demandeurs proposent une solution consistant en un film de polymère permettant aux cations Pb ou Zn d'imprégner une matrice solide destinée à servir de couche sensible et compatible de l'élaboration d'un capteur passif à onde élastique.

La présente invention a ainsi pour objet un capteur de H₂S, comprenant au moins un transducteur à onde élastique et un film comportant une matrice de polymère comprenant des fonctions carboxylates et des cations de plomb ou de zinc.

Le film représente la couche sensible du capteur.

Selon des variantes, le capteur comporte une antenne permettant d'interroger ledit capteur à distance et ainsi réaliser un capteur passif interrogeable à distance.

Le film de polymère peut présenter typiquement une épaisseur comprise entre une centaine de nanomètres et quelques microns.

Selon des variantes de l'invention, le polymère répond aux formules chimiques suivantes : 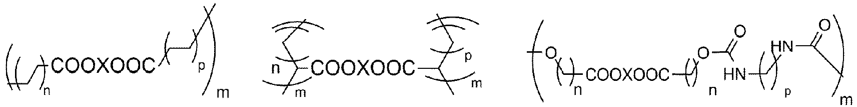 avec X = Pb ou Zn
avec n, m et p des nombres entiers.

Pour obtenir la couche sensible de film de polymère, les Demandeurs ont développé des polymères incorporant les fonctions nécessaires, c'est-à-dire incorporant du plomb ou du zinc et des fonctions carboxylates et plus précisément par exemple pour cela, des familles de molécules du type : *(R₁COO)X(R₂COO) où R₁ et R₂* désignent des groupements possédant des fonctions réactives qui permettent de connecter les cations Pb(ll) ou les cations Zn(ll) par ses ligands dans des macromolécules.

Afin de maximiser la sensibilité de la couche organique, il est intéressant de maximiser la densité d'ions Pb(ll) ou d'ions Zn(ll) dans la matrice en réduisant de préférence autant que possible la longueur des chaînes carbonées des monomères.

Les Demandeurs sont partis du constat que la fonctionnalisation doit d'une part affecter le moins possible la propagation de l'onde élastique en absence de H₂S mais également offrir suffisamment de sites réactifs pour induire une variation significative par exemple de la célérité de l'onde élastique en présence de H₂S.

Ce compromis ainsi que le contrôle de l'épaisseur de la couche et la densité des sites récepteurs, peuvent être optimisés. Ainsi afin de limiter d'une part la perturbation induite par le film de polymère sur la propagation de l'onde élastique, tout en maximisant d'autre part la variation de la célérité de l'onde par variation des conditions limites de propagation, il est particulièrement intéressant d'utiliser une épaisseur de film de l'ordre de 1 à 5% de la longueur d'onde élastique. Pour une fréquence de travail de 150 MHz par exemple, une telle condition conduit à une épaisseur de film comprise entre environ 250 nm et 5 µm.

Il est à noter en effet, que les mêmes gammes d'épaisseur peuvent être employées qu'il s'agisse de transducteur à onde de surface ou de transducteur à onde de volume. L'épaisseur variant comme l'inverse de la racine de la fréquence, passer d'un résonateur à onde de volume ( fréquence de travail typiquement de l'ordre de 5-10 MHz) à un transducteur à onde élastique (fréquence de travail typiquement de l'ordre du 1 GHz) ne fait varier l'épaisseur de film que d'un facteur 10.

Selon des variantes de l'invention, le transducteur est un transducteur à onde élastique de surface.

Selon des variantes de l'invention, le capteur comporte une ligne à retard comportant au moins un transducteur à onde élastique de surface et un miroir, la couche sensible étant positionnée entre ledit transducteur et ledit miroir. Avantageusement, la ligne à retard comprend un transducteur positionné entre deux miroirs, ladite couche sensible étant positionnée entre ledit transducteur et l'un desdits miroirs.

Selon des variantes de l'invention, le transducteur est un transducteur à onde de volume ou de Lamb.

Avantageusement, le transducteur incorporé au capteur est un dipôle, qu'il comprenne une architecture de ligne à retard ou un résonateur propageant une onde élastique.

Selon des variantes de l'invention, le transducteur comprend un substrat en niobate de lithium ou en tantalate de lithium.

L'invention a aussi pour objet un procédé de fabrication d'un capteur selon l'invention, comprenant les étapes suivantes :
- le dépôt à la surface d'un substrat, de monomère contenant un complexe de carboxylate de plomb ou de zinc et une fonction polymérisable, pouvant être, par exemple, de formule chimique : 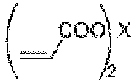 avec X : Pb²⁺ ou Zn²⁺
- la polymérisation dudit monomère.

L'invention a encore pour objet un procédé de fabrication d'un capteur selon l'invention comprenant le dépôt sur un substrat d'un film de polymère, préalablement synthétisé pouvant être de formule chimique : 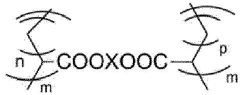 avec X : Pb²⁺ ou Zn²⁺
avec n,m et p entiers.

L'invention a également pour objet un procédé de fabrication d'un capteur selon l'invention comprenant le dépôt sur un substrat d'un film de polymère obtenu par polycondensation ou polyaddition ou polyélimination entre un monomère contenant un complexe de carboxylate de plomb ou de zinc et une molécule difonctionnelle. Le polymère final peut être de formule chimique : 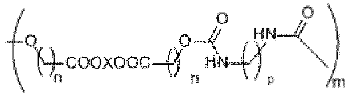 avec X : Pb²⁺ ou Zn²⁺
avec n, m et p entiers.

L'invention a encore pour objet un dispositif de détection de H₂S comprenant :
- un capteur selon l'invention ;
- des moyens d'interrogation radiofréquence ;
- des moyens de détection de la réponse dudit capteur.

Selon des variantes de l'invention, le dispositif comprend des moyens d'analyse de la célérité ou des pertes d'insertion de l'onde élastique pour identifier la présence du composé H₂S.

L'invention sera mieux comprise et d'autres avantages apparaîtront à la lecture de la description qui va suivre donnée à titre non limitatif et grâce aux figures parmi lesquelles :
- les figures 1a et 1b illustrent un exemple de capteur à onde élastique de surface selon la présente invention et une configuration de test utilisant des transducteurs à onde élastique de surface ;
- les figures 2a et 2b illustrent des exemples de capteur à onde de volume selon la présente invention ;
- la figure 3 illustre une architecture de lecteur de capteur selon l'invention interrogeable à distance ;
- les figures 4a et 4b illustrent des courbes de réponse en termes de magnitude et de phase d'un capteur selon l'invention, en fonction de la fréquence d'interrogation (avant et après fonctionnalisation par du H₂S de la couche sensible).

Le capteur de H₂S selon l'invention peut être un capteur filaire ou un capteur passif interrogeable à distance.

Le film sensible à H₂S peut avantageusement être ainsi combiné à un transducteur à onde élastique pour réaliser un capteur passif interrogeable à distance selon l'invention, le transducteur à onde élastique étant capable de convertir un signal électromagnétique en vibration mécanique.

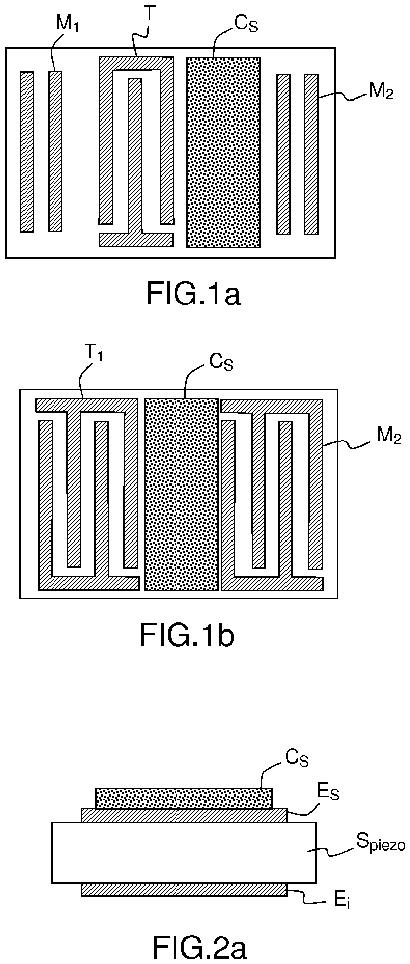

La figure 1a illustre un exemple de capteur à onde élastique de surface de l'invention, de configuration compacte.

Le capteur comporte une ligne à retard réflective, présentant deux miroirs M₁ et M₂, un transducteur T situé entre les deux miroirs.

Il peut être particulièrement intéressant que le capteur intègre un transducteur à onde élastique relié à une antenne et plusieurs miroirs, permettant ainsi d'effectuer une mesure multiparamètres tel que décrite dans l'article : J. Micro/Nanolith. MEMS MOEMS 8(3) 031306 (2009) - Wireless surface acoustic wave chemical sensor for simultaneous measurement of CO2 and humidity.

Typiquement le transducteur peut comporter de manière classique des électrodes interdigitées.

La présence de deux miroirs permet d'avoir une zone de référence, sans laquelle la mesure de H₂S est polluée par la variation de distance lecteur-capteur.

Il est donc utile de disposer d'une zone sensible pour la mesure, et d'une zone libre de fonctionnalisation de surface pour établir la distance RADAR-capteur.

Une couche Cs de film de polymère sensible au H₂S est déposée entre le transducteur et un des miroirs Plus précisément dans cette architecture miroir-transducteur-miroir, la zone séparant le premier miroir du transducteur est libre de toute fonctionnalisation de surface pour établir le temps de vol en espace libre de l'onde élastique (quelle que soit la concentration de gaz), tandis que la seconde région entre le transducteur et le second miroir est fonctionnalisée pour faire varier la célérité de l'onde élastique avec l'absorption du gaz.

Pour une mesure sans fil dans une configuration de ligne à retard réflective, des substrats fortement couplés (coefficient de couplage supérieur à 1 %), tels que le niobate de lithium ou le tantalate de lithium sont favorisés.

La figure 1b illustre quant à elle, une configuration simple comportant deux transducteurs à onde élastique de surface T₁ et T₂, entre lesquels est positionnée une couche sensible Cs de film de polymère. Une telle configuration a été utilisée par le Demandeur pour valider expérimentalement la sensibilité du film mince de polymère utilisé dans la présente invention à la détection de H₂S, comme il sera explicité ultérieurement.

Le transducteur employé dans le capteur passif de l'invention peut également être un transducteur à onde de volume ou de Lamb, les transducteurs à onde de surface étant tout de même plus avantageux en termes de montée en fréquence nécessaire à la réduction d'antennes de l'émetteur et du récepteur.

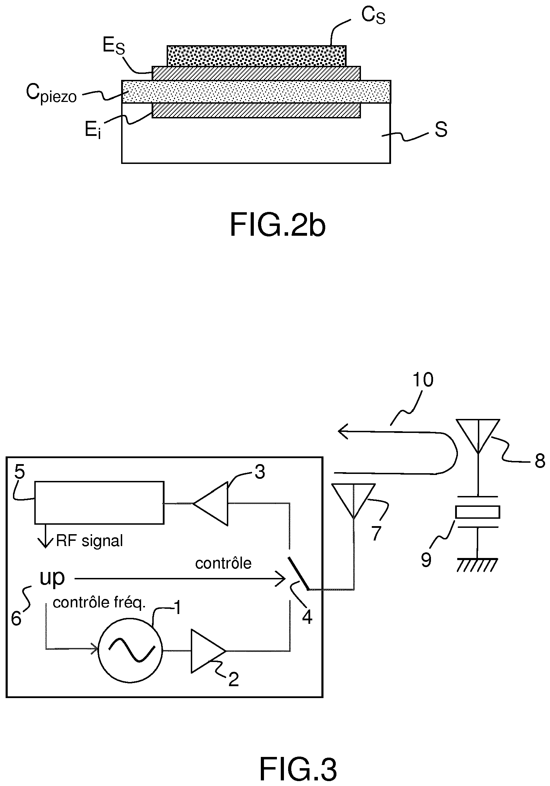

Des variantes de transducteurs à onde de volume sont représentées en figures 2a et 2b.

Ces transducteurs peuvent être de géométrie à symétrie cylindrique.

La figure 2a illustre une première configuration présentant une électrode Es recouverte d'une couche sensible Cs, un substrat piézoélectrique S_{piézo} étant inserré entre l'électrode supérieure Es et une électrode inférieure Ei.

La figure 2b illustre une seconde configuration présentant un substrat piézoélectrique fin C_{piézo} déposé sur un substrat S présentant de faibles pertes élastiques, deux électrodes Es et Ei (électrode enterrée) entourent le substrat fin, de manière à constituer le transducteur à onde de volume. La couche sensible Cs est déposée à la surface de l'électrode supérieure Es.

Le capteur ainsi réalisé peut être interrogé à distance, et la réponse est porteuse de l'information de détection de présence de H₂S.

La figure 3, illustre à ce titre un exemple d'architecture typique de lecteur de transducteur à onde élastique interrogé par une liaison sans fil sur porteuse radiofréquence.

Une porteuse est générée par une source 1 et amplifiée par un amplificateur 2 avant d'être émise au travers d'un commutateur 4.

Une impulsion est formée en commutant le commutateur entre la source et l'étage de réception formé d'un amplificateur à faible bruit 3 et d'un détecteur 5 de phase et de magnitude (I/Q).

Une antenne 7 connectée au point commun 4 rayonne le signal 10 qui téléalimente, au travers d'une antenne 8, le capteur passif 9 comportant le film sensible à H₂S. Sur la figure 3, le capteur passif est schématisé par une représentation de résonateur (même dans le cas de l'utilisation d'une ligne à retard).

La réponse du capteur 9 porte la signature de la détection du composé chimique H₂S, qu'il s'agisse de temps de vol de l'onde élastique (dans le cas d'une ligne à retard) ou d'une variation de fréquence de résonance, dans le cas d'un capteur comportant un résonateur.

La référence up est relative à un exemple de microcontrôleur, un STM32F103, mais le type exact de microcontrôleur n'a pas d'incidence, ce microcontrôleur a pour fonction de séquencer les diverses étapes de mesures, sans contrainte de puissance de calcul. Il pourrait s'agir d'un composant FPGA, d'un composant DSP, ou de tout composant numérique programmable capable de séquencer les mesures.

Pour obtenir la couche sensible constituée du film de polymère, les Demandeurs ont ainsi développé des polymères incorporant les fonctions nécessaires. Ils ont développé notamment pour cela, des familles de molécules du type : *(R₁COO)X(R₂COO) où R₁ et R₂* désignent des groupements possédant des fonctions réactives qui permettent de connecter les cations par ses ligands dans des macromolécules, et X un cation Pb²⁺ ou un cation Zn²⁺ .

Plusieurs exemples de synthèse de polymères utilisés dans la présente invention sont présentés ci-après :

### Premier exemple de synthèse :

La première synthèse est fondée sur la formation d'un complexe de carboxylate de plomb(II) ou zinc(II) contenant une fonction polymérisable constituant un monomère, soluble, qui peut être déposé sous la forme d'un film mince sur un substrat.

Puis, ces monomères sont polymérisés sous l'effet d'un stimulus extérieur (chaleur, photons) pour obtenir un film réticulé. 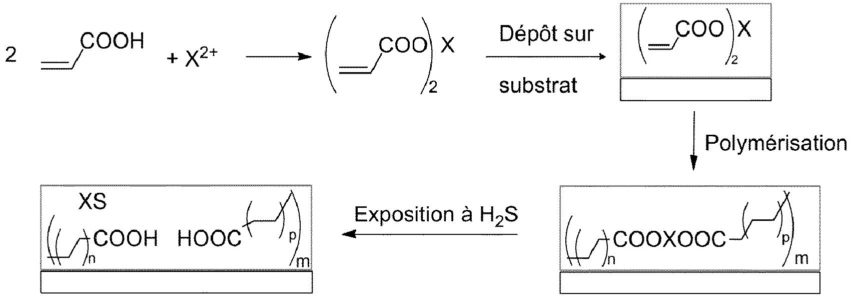 avec X : Pb²⁺ ou Zn²⁺

### Deuxième exemple de synthèse :

Cette seconde synthèse est fondée sur la complexation de cations Pb(ll) ou Zn(ll) par un polymère contenant des fonctions acides carboxyliques pendantes. Ensuite, le polymère est déposé sous la forme d'un film mince sur un substrat :

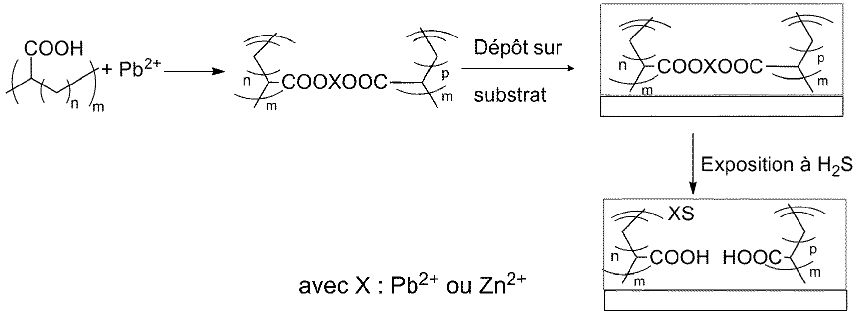

### Troisième exemple de synthèse :

Ce troisième exemple de synthèse est fondée sur la complexation de cations Pb(II) ou Zn(II) par des molécules polyfonctionnelles qui peuvent ensuite réagir par polycondensation, polyaddition ou polyélimination (ou toutes autres combinaisons de ces réactions) avec des molécules polyfonctionnelles complémentaires pour former un film mince polymère sur un substrat selon, par exemple le schéma réactionnel suivant :

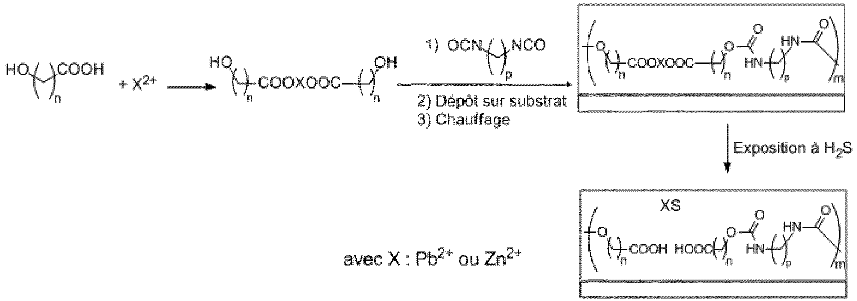

Dans les différents cas, les films minces soumis ensuite à l'exposition à H₂S gazeux peuvent générer la formation de sulfure de plomb(II) ou zinc(II), *i.e.* PbS ou ZnS.

Dans le cadre de la présente invention, la formation de PbS ou ZnS conduit à une variation de masse accompagnée d'un changement de couleur.

La variation relative de masse, qui est le paramètre essentiel dans la détection acoustique, dépend directement du choix des précurseurs organiques de la matrice.

Si le monomère de départ est de l'acide acrylique ; sa masse molaire est de 72 g/mol. La masse molaire du plomb étant de 207 g/mol, la complexation de la couche sensible par H₂S (masse molaire 34g/mol) conduit à une variation de masse relative de 34 / (207 + 72×2) = 10%.

Il est à noter que de manière générale, un transducteur à onde acoustique de surface est connu pour être capable de détecter une variation de masse de l'ordre de quelques dizaines de ng/cm², définissant la limite de détection accessible au moyen de ce type de transducteur.

Les Demandeurs ont procédé à des essais permettant de valider le concept de capteur de la présente invention et le choix de la couche sensible permettant effectivement une détection de H₂S, dans un capteur passif à onde élastique pouvant être interrogé à distance.

Cette démonstration expérimentale de concept de capteur repose sur quatre étapes :
Etape1 : la synthèse et la caractérisation des molécules sensibles à H₂S. La sensibilité à H₂S est testée par une exposition directe des molécules à H₂S déclenchant un changement de couleur ;
Etape 2 : l'élaboration d'un film mince transparent sur une lame de verre et l'étude de la polymérisation des molécules en phase solide ;
Etape 3 : la détection d'un changement d'absorbance de ce film mince lors d'une exposition à H₂S ;
Etape 4 : le dépôt d'un film mince sur un transducteur à onde élastique de surface pour estimer la variation de la célérité de l'onde élastique lors de l'exposition des molécules à H₂S.

Dans un premier temps, des complexes de plomb(II) ont été synthétisés et caractérisés par les méthodes standard de la chimie analytique, permettant d'élaborer plusieurs grammes de molécules à chaque synthèse.

Le H₂S qui est utilisé pour les tests est produit par un traitement acide d'un sel de soufre. Le premier essai a consisté en l'exposition de la poudre de complexes de plomb(II) à un flux de H₂S gazeux dans des conditions normales.

La réaction apparaît dans un intervalle de temps inférieur à la durée entre deux photographies extraites d'un film à 50 images/seconde, quasi-instantanée et probablement limitée par le temps de diffusion du gaz. Le changement de couleur - d'une poudre blanche à une poudre noire - est visible sur l'échantillon de poudre situé au fond du récipient.

Ayant validé la réactivité du composé synthétisé avec H₂S, le dépôt de la molécule solubilisée dans le diméthylsulfoxyde (DMSO) est obtenu par évaporation du solvant couramment dénommé « drop casting ».

La qualité de la polymérisation est validée par la tenue mécanique du film.

Une fois le film formé sur lame de verre, le changement de couleur valide également la réactivité du film lors de l'exposition à H₂S. Les demandeurs ont validé l'obtention de films minces de polymères par spin coating sur des substrats en silicium. Les échantillons utilisés présentent des surfaces de l'ordre de 5 à 10 cm². Les jeux de paramètres (solvant, viscosité, température, vitesse de rotation) ont été optimisés afin de déterminer des protocoles robustes et reproductibles pour obtenir des couches minces d'épaisseur contrôlées entre 0,5 et 2 microns. Puis, les demandeurs ont vérifié que ces films étaient toujours sensibles à la présence d'H₂S. Lorsque ces films sont exposés à H₂S, un changement de couleur est visible.

Enfin, la procédure de dépôt est reproduite sur un transducteur à onde élastique de surface en transmission (substrat de quartz propageant une onde de Love). Un tel dispositif ne propage pas la même nature d'onde qu'une ligne à retard réflective sur niobate de lithium qui peut avantageusement être utilisée pour une mesure radiofréquence sans fil, mais son comportement est convenablement maîtrisé et permet une analyse fine du comportement du film mince organique soumis à H₂S.

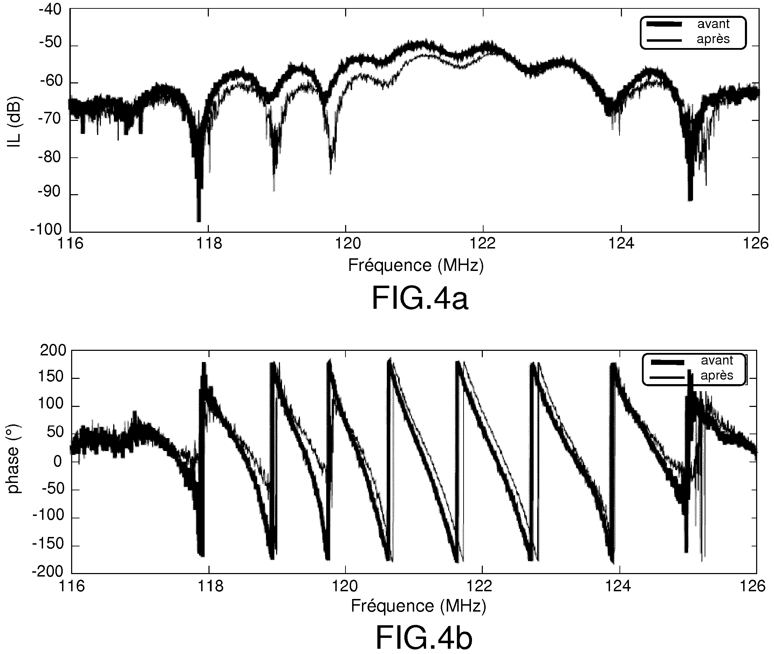

Les figures 4a et 4b illustrent les résultats des mesures de détermination de la magnitude IL et de la phase en fonction de la fréquence d'interrogation et ce avant fonctionnalisation et après fonctionnalisation du polymère, résultat de l'exposition au H₂S (avant fonctionnalisation, chaque ligne à retard présente une atténuation de l'ordre de 23 dB. Le dépôt du film induit une atténuation significative dépendante de l'épaisseur du film) et l'évolution de la phase du signal.

Les courbes en traits gras sont relatives à ces paramètres mesurés avant réaction avec du H₂S, les courbes en traits fins étant relatives à ces paramètres obtenus après réaction.

L'évolution de la phase vers les hautes fréquences est a priori surprenante car une augmentation de la masse du film se traduit généralement par un décalage des courbes vers les basses fréquences.

Dans le cas d'une élévation de la célérité de l'onde élastique (observée comme décalage de la courbe vers les hautes fréquences), l'interprétation classique est d'attribuer cette élévation de célérité à une rigidification de la couche organique adsorbée.

Les Demandeurs attribuent cet effet au passage du plomb sous forme d'ions dans la matrice, à une forme de nanoparticules de PbS dans la matrice organique.

Ce comportement (augmentation de la célérité) est relativement inhabituel en détection de composés gazeux par une couche mince organique.

Un intérêt majeur de cette observation est de fournir une distinction claire entre une détection de H₂S et l'interférence par de l'absorption non-spécifique d'espèces chimiques autres présentes dans l'atmosphère : ces dernières induisant une variation de masse du film - sans le rigidifier - qui se traduit par une baisse de la célérité de l'onde élastique (ou une décroissance de la phase si le transducteur est sondé à fréquence fixe).

Le risque de faux positif est donc réduit par cette analyse.

La variation de près de 20° de la phase lors de l'exposition à H₂S est significative, et considérée comme constituant un signal très facilement détectable. Pour référence, l'article de D. Rabus, J.-M. Friedt, S. Ballandras, G. Martin, E . Carry, and V. Blondeau-Patissier. A high sensitivity open loop electronics for gravimetric acoustic wave-based sensors. IEEE Trans. UFFC, 60(6) :1219-1226, 2013 démontre une limite de détection plus de 500 fois plus faible (dans un contexte de ligne à retard en transmission).

## Revendications

1. Capteur de H₂S, comprenant au moins un transducteur à onde élastique et **caractérisé en ce qu'**il comprend un film comportant une matrice de polymère comprenant des fonctions carboxylates et des cations de plomb ou de zinc.

2. Capteur selon la revendication 1, **caractérisé en ce qu'**il comporte une antenne permettant d'interroger ledit capteur à distance.

3. Capteur selon l'une des revendications 1 ou 2, dans lequel le film présente une épaisseur comprise entre une centaine de nanomètres et quelques microns.

4. Capteur selon l'une des revendications 1 à 3, dans lequel le polymère répond à la formule chimique suivante : 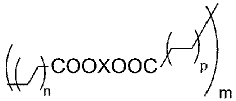 avec X : Pb²⁺ ou Zn²⁺
avec n, m et p des nombres entiers.

5. Capteur selon l'une des revendications 1 à 3, dans lequel le polymère répond à la formule chimique suivante : 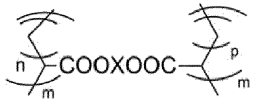 avec X : Pb²⁺ ou Zn²⁺
avec n, m et p des nombres entiers.

6. Capteur selon l'une des revendications 1 à 3, dans lequel le polymère répond à la formule chimique suivante : 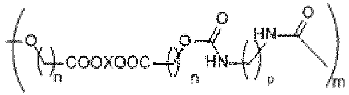 avec X : Pb²⁺ ou Zn²⁺
avec n, m et p des nombres entiers.

7. Capteur selon l'une des revendications 1 à 6, dans lequel le film présente une épaisseur comprise entre 1 à 5% de la longueur d'onde élastique.

8. Capteur selon la revendication 7, dans lequel le film présente une épaisseur comprise entre 250 nm et 5 µm.

9. Capteur selon l'une des revendications 1 à 8, **caractérisé en ce que** le transducteur est un transducteur à onde élastique de surface.

10. Capteur selon la revendication 9, **caractérisé en ce qu'**il comporte une ligne à retard comportant au moins un transducteur à onde élastique de surface et un miroir.

11. Capteur selon la revendication 9, **caractérisé en ce qu'**il comprend un transducteur positionné entre deux miroirs, ladite couche sensible étant positionnée entre ledit transducteur et l'un desdits miroirs.

12. Capteur selon l'une des revendications 1 à 8, **caractérisé en ce que** le transducteur est un transducteur à onde élastique de volume ou de Lamb.

13. Capteur selon l'une des revendications 1 à 12, **caractérisé en ce que** le transducteur comprend un substrat en niobate de lithium ou en tantalate de lithium.

14. Procédé de fabrication d'un capteur selon l'une des revendications 1 à 13, comprenant la réalisation dudit film de polymère qui comporte les étapes suivantes :
- le dépôt à la surface d'un substrat, de monomère formé d'un complexe de carboxylate de plomb ou de zinc et une fonction polymérisable, pouvant être de formule chimique : 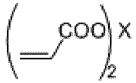 avec X : Pb²⁺ ou Zn²⁺
la polymérisation dudit monomère.

15. Procédé de fabrication d'un capteur selon l'une des revendications 1 à 13 comprenant :
- la synthèse d'un polymère de formule chimique : 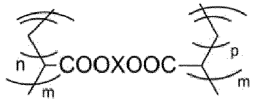 avec X : Pb²⁺ ou Zn²⁺
avec n, m et p entiers ;
- le dépôt sur un substrat d'un film dudit polymère préalablement synthétisé.

16. Procédé de fabrication d'un capteur selon l'une des revendications 1 à 13 comprenant :
- la synthèse d'un polymère est de formule chimique 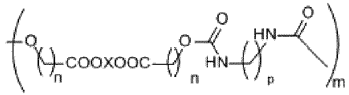 avec X : Pb²⁺ ou Zn²⁺
avec n, m et p entiers ; ladite synthèse étant réalisée par polycondensation ou polyaddition ou polyélimination d'un monomère contenant un complexe de carboxylate de plomb ou de zinc et une molécule difonctionnelle ;
- le dépôt sur un substrat d'un film dudit polymère préalablement synthétisé.

17. Dispositif de détection de H₂S comprenant :
- un capteur selon l'une des revendications 1 à 13 ;
- des moyens d'interrogation radiofréquence ;
- des moyens de détection de la réponse dudit capteur.

18. Dispositif selon la revendication 17, **caractérisé en ce qu'**il comprend des moyens d'analyse de la célérité ou des pertes d'insertion de l'onde élastique pour identifier la présence du composé H₂S.

## Patentansprüche

1. H₂S-Sensor, der mindestens einen Wandler für elastische Wellen umfasst und **dadurch gekennzeichnet ist, dass** er einen Film mit einer Polymermatrix umfasst, die Carboxylatfunktionen und Blei- oder Zinkkationen umfasst.

2. Sensor nach Anspruch 1, **dadurch gekennzeichnet, dass** er eine Antenne umfasst, die den Sensor fernabfragen kann.

3. Sensor nach einem der Ansprüche 1 oder 2, wobei der Film eine Dicke zwischen etwa hundert Nanometern und einigen Mikrometern aufweist.

4. Sensor nach einem der Ansprüche 1 bis 3, wobei der Polymer der folgenden chemischen Formel entspricht: 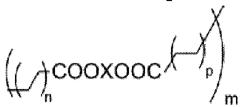 mit X: Pb²⁺ oder Zn²⁺
wobei n, m und p ganze Zahlen sind.

5. Sensor nach einem der Ansprüche 1 bis 3, wobei das Polymer der folgenden chemischen Formel entspricht: 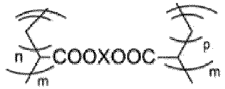 mit X: Pb²⁺ oder Zn²⁺
wobei n, m und p ganze Zahlen sind.

6. Sensor nach einem der Ansprüche 1 bis 3, wobei das Polymer der folgenden chemischen Formel entspricht: 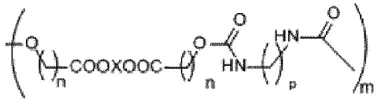 mit X: Pb²⁺ oder Zn²⁺
wobei n, m und p ganze Zahlen sind.

7. Sensor nach einem der Ansprüche 1 bis 6, wobei der Film eine Dicke von 1 bis 5 % der elastischen Wellenlänge aufweist.

8. Sensor nach Anspruch 7, wobei der Film eine Dicke zwischen 250 nm und 5 µm aufweist.

9. Sensor nach einem der Ansprüche 1 bis 8, **dadurch gekennzeichnet, dass** der Wandler ein Wandler für elastische Oberflächenwellen ist.

10. Sensor nach Anspruch 9, **dadurch gekennzeichnet, dass** er eine Verzögerungsleitung mit mindestens einem Wandler für elastische Oberflächenwellen und einen Spiegel umfasst.

11. Sensor nach Anspruch 9, **dadurch gekennzeichnet, dass** er einen zwischen zwei Spiegeln positionierten Wandler umfasst, wobei die empfindliche Schicht zwischen dem Wandler und einem der Spiegel positioniert ist.

12. Sensor nach einem der Ansprüche 1 bis 8, **dadurch gekennzeichnet, dass** der Wandler ein Wandler für volumenelastische oder Lamb-Wellen ist.

13. Sensor nach einem der Ansprüche 1 bis 12, **dadurch gekennzeichnet, dass** der Wandler ein Lithiumniobat- oder Lithiumtantalat-Substrat umfasst.

14. Verfahren zur Herstellung eines Sensors nach einem der Ansprüche 1 bis 13, das die Herstellung des Polymerfilms umfasst, das die folgenden Schritte beinhaltet:
- Absetzen, auf die Oberfläche eines Substrats, eines Monomers, gebildet aus einem aus Blei- oder Zinkcarboxylat und einer polymerisierbaren Funktion gebildeten Komplex, das die folgende chemische Formel haben kann: 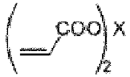 mit X: Pb²⁺ oder Zn²⁺
Polymerisieren des Monomers.

15. Verfahren zur Herstellung eines Sensors nach einem der Ansprüche 1 bis 13, das Folgendes umfasst:
- Synthetisieren eines Polymers der chemischen Formel : 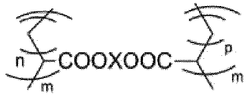 mit X: Pb²⁺ oder Zn²⁺
wobei n, m und p ganze Zahlen sind;
- Absetzen eines Films aus dem zuvor synthetisierten Polymer auf ein Substrat.

16. Verfahren zur Herstellung eines Sensors nach einem der Ansprüche 1 bis 13, das Folgendes umfasst:
- Synthetisieren eines Polymers der chemischen Formel 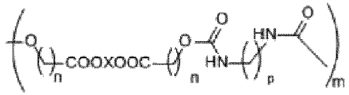 mit X: Pb²⁺ oder Zn²⁺
wobei n, m und p ganze Zahlen sind:
wobei das Synthetisieren durch Polykondensation oder Polyaddition oder Polyelimination eines Monomers erfolgt, das einen Komplex aus Blei- oder Zinkcarboxylat und ein bifunktionelles Molekül enthält;
- Absetzen eines Films aus dem zuvor synthetisierten Polymer auf ein Substrat.

17. Gerät zum Nachweisen von H₂S, das Folgendes umfasst:
- einen Sensor nach einem der Ansprüche 1 bis 13;
- Funkfrequenz-Abfragemittel;
- Mittel zum Erfassen der Reaktion des Sensors.

18. Gerät nach Anspruch 17, **dadurch gekennzeichnet, dass** es Mittel zum Analysieren der Schnelligkeit oder von Einfügungsverlusten der elastischen Welle umfasst, um das Vorliegen der H₂S-Verbindung zu identifizieren.

## Claims

1. H₂S sensor, comprising at least one elastic wave transducer and **characterized in that** it comprises a film having a polymer matrix comprising carboxylate functional groups and lead or zinc cations.

2. Sensor according to claim 1, **characterized in that** it comprises an antenna which makes it possible to remotely interrogate said sensor.

3. Sensor according to one of claims 1 or 2, wherein the film exhibits a thickness of between about a hundred nanometres and a few microns.

4. Sensor according to one of claims 1 to 3, wherein the polymer corresponds to the following chemical formula: 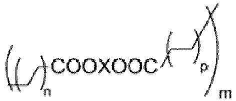 with X : Pb²⁺ or Zn²⁺
with n, m and p integers.

5. Sensor according to one of claims 1 to 3, wherein the polymer corresponds to the following chemical formula: 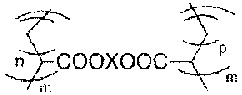 with X: Pb²⁺ or Zn²⁺
with n, m and p integers.

6. Sensor according to one of claims 1 to 3, wherein the polymer corresponds to the following chemical formula: 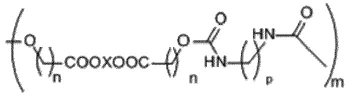 with X : Pb²⁺ or Zn²⁺
with n, m and p integers.

7. Sensor according to one of claims 1 to 6, wherein the film exhibits a thickness of between 1 and 5% of the elastic wavelength.

8. Sensor according to claim 7, wherein the film exhibits a thickness of between 250 nm and 5 µm.

9. Sensor according to one of claims 1 to 8, **characterized in that** the transducer is a surface elastic wave transducer.

10. Sensor according to Claim 9, **characterized in that** it comprises a delay line having at least one surface elastic wave transducer and a mirror.

11. Sensor according to claim 9, **characterized in that** it comprises a transducer positioned between two mirrors, said sensitive layer being positioned between said transducer and one of said mirrors.

12. Sensor according to one of claims 1 to 8, **characterized in that** the transducer is a volume elastic wave or Lamb elastic wave transducer.

13. Sensor according to one of claims 1 to 12, **characterized in that** the transducer comprises a substrate made of lithium niobate or made of lithium tantalate.

14. Process for the manufacture of a sensor according to one of claims 1 to 13, comprising the preparation of said polymer film which includes the following steps:
- the deposition at the surface of a substrate, of a monomer formed of a lead or zinc carboxylate complex and a polymerizable functional group, which can be of chemical formula: 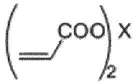 with X : Pb²⁺ or Zn²⁺
the polymerization of the said monomer.

15. Process for the manufacture of a sensor according to one of claims 1 to 13, comprising:
- the synthesis of a polymer of chemical formula: 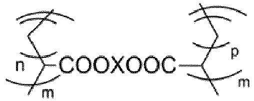 with X : Pb²⁺ or Zn²⁺
with n, m and p integers;
- the deposition on a substrate of a film of said polymer synthesized beforehand.

16. Process for the manufacture of a sensor according to one of claims 1 to 13, comprising:
- the synthesis of a polymer of chemical formula: 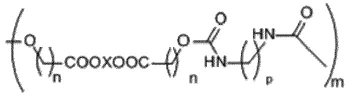 with X : Pb²⁺ or Zn²⁺
with n, m and p integers;
said synthesis being carried out by polycondensation or polyaddition or polyelimination of a monomer containing a lead or zinc carboxylate complex and a difunctional molecule;
- the deposition on a substrate of a film of said polymer synthesized beforehand.

17. H₂S detection device comprising:
- a sensor according to one of claims 1 to 13;
- radiofrequency interrogation means;
- means for detecting the response of said sensor.

18. Device according to Claim 17, **characterized in that** it comprises means for analysis of the speed or of the insertion losses of the elastic wave in order to identify the presence of the compound H₂S.
